# EUROPEAN PATENT SPECIFICATION

(11) **EP 3 251 636 B1**
(45) Date of publication and mention of the grant of the patent: **15.09.2021**
(21) Application number: 16882802.8
(22) Date of filing: 11.05.2016
(51) Int. Cl.: A61B 10/00, A61B 5/00

(54) **PHYSIOLOGICAL REGULATING SYSTEM AND SMART UNDERPANTS**
PHYSIOLOGISCHES EINSTELLSYSTEM UND INTELLIGENTE UNTERHOSEN
SYSTÈME DE RÉGULATION PHYSIOLOGIQUE ET SOUS-VÊTEMENTS INTELLIGENTS

(30) Priority: 21.03.2016 CN 201610161082
(43) Date of publication of application: 06.12.2017
(73) Proprietor: BOE Technology Group Co., Ltd., Beijing 100015 (CN); Boe Optical Science and Technology Co., Ltd., Suzhou Industrial Park Suzhou Jiangsu 215000 (CN)
(72) Inventor: ZHU, Ling, Beijing 100176 (CN)
(74) Representative: AWA Sweden AB
(86) International application number: PCT/CN2016/081670
(87) International publication number: WO 2017/161648

(56) References cited:
- EP-A2- 0 090 327
- WO-A1-00/65096
- WO-A1-2016/035073
- CN-A- 104 055 618
- CN-U- 202 980 154
- CN-U- 204 337 118
- CN-U- 204 723 036
- CN-U- 204 723 098
- JP-A- 2005 164 405
- US-A1- 2014 206 947

## Description

### BACKGROUND

### Technical Field

Embodiments of the present disclosure relate to a field of physiological adjustment, and more particularly, to a physiological adjustment system and intelligent underpants.

### Description of the Related Art

Most of existing underclothes only have an ordinary function, i.e., functioning as clothes, but cannot adjust physiology of a human body according to physiological state information of the human body. Especially for women, in the early menstrual period and during the whole menstrual period, some symptoms such as cold uterus, dysmenorrhea and the like occur due to a low uterus temperature, which makes a majority of women miserable and even seriously affects daily work and daily life of the women.

Therefore, it is very urgent to provide a device and a method capable of monitoring the physiological state information of the human body and adjusting the physiology of the human body.

WO 2016/035073 discloses apparatus and methods including a sensor configured to monitor a subject and to generate a sensor signal in response thereto. A control unit analyzes the sensor signal, and controls a property of a sound signal, in response to (a) the analyzing of the sensor signal, and (b) a historical physiological parameter of the subject that was exhibited in response to a historical sound signal. The control unit drives a speaker to play the sound signal.

CN 204 337 118 (utility model) discloses dysmenorrhea therapeutic equipment which comprises underpants and a waistband.

EP 0 090 327 discloses a device for determining ovulation in women.

US 2014/206947 discloses a treatment device having a heat source, a power source, a heat applicator and a lighting mechanism.

### SUMMARY

One of the objects of the present disclosure is to provide a physiological adjustment system capable of monitoring physiological state information of a human body and heating the body. Another object of the present disclosure is to provide intelligent underpants comprising a physiological adjustment system. A further non-claimed object of the present disclosure is to provide a physiological adjustment method.

According to an embodiment of the present disclosure, there is provided a physiological adjustment system, comprising the features of independent claim 1. Preferred embodiments are defined in the dependent claims.

In a non-limiting embodiment, the physiological state information comprises second physiological state information different from the first physiological state information, the first heating command is generated based on the second physiological state information, and the control module is further configured for generating a physiological result based on the second physiological state information and determining whether to generate the first heating command based on the physiological result.

In a non-limiting embodiment, the second physiological state information comprises state information regarding whether a woman is in an ovulation period, the physiological result comprises woman menstrual period information, and the control module is further configured for generating the woman menstrual period information based on the state information regarding whether the woman is in the ovulation period and determining whether to generate the first heating command based on the woman menstrual period information.

In a non-limiting embodiment, the physiological adjustment system further comprises a communication module for receiving a command from an external decision device and transmitting the physiological state information detected by the detection module to the external decision device, wherein the external decision device is configured for generating a start command based on the transmitted physiological state information, and the control module is configured for receiving the start command and determining whether to generate a second heating command based on the start command.

In a non-limiting embodiment, the control module is configured for generating only one of the first heating command and the second heating command in a preset period of time.

In a non-limiting embodiment, the external decision device comprises an intelligent terminal comprising an application program for generating the start command based on the transmitted physiological state information.

In a non-limiting embodiment, the communication module comprises a wireless communication module.

In a non-limiting embodiment, the physiological state information transmitted by the communication module comprises state information regarding whether a woman is in a menstrual period and/or state information regarding whether a woman is in an ovulation period, and
the control module is configured for determining whether to generate the start command based on the state information regarding whether the woman is in the menstrual period; and/or
the control module is configured for generating woman menstrual period information based on the state information regarding whether the woman is in the ovulation period and for determining whether to generate the start command based on the woman menstrual period information.

In a non-limiting embodiment, the physiological adjustment system further comprises a flexible display capable of displaying at least one kind of information from an information group consisting of information regarding whether a woman is in a menstrual period, a body temperature, and a mood sign indicating physiological state information of the woman and information regarding whether the woman is in an ovulation period.

In a non-limiting embodiment, the at least one kind of information from the information group is transmitted to an external intelligent terminal by the communication module.

In a non-limiting embodiment, the control module comprises a manual heating switch, and the heating command further comprises a third heating command generated by the manual heating switch.

According to another embodiment of the present disclosure, there are provided intelligent underpants, comprising the physiological adjustment system according to the above embodiments, wherein the heating module is arranged at a position corresponding to a lower abdomen of a wearer on a front side of the underpants.

In a non-limiting embodiment, the intelligent underpants further comprise an insulating bag filled with essential oil, and a heat generation source of the heating module is provided in the essential oil.

In a non-limiting embodiment, the detection module comprises a blood sensor for detecting blood to obtain state information regarding whether a woman is in a menstrual period.

In a non-limiting embodiment, the detection module comprises an electronic ovulation test strip sensor provided in a crotch portion of the intelligent underpants, and the electronic ovulation test strip sensor is configured for detecting a peak level of luteinizing hormone in residual urine to obtain state information regarding whether a woman is in an ovulation period.

In a non-limiting embodiment, the physiological adjustment system further comprises a temperature sensor for measuring an abdomen temperature of the wearer.

According to a further non-claimed example of the present disclosure, there is provided a physiological adjustment method, comprising steps of:
detecting a physiological state to obtain physiological state information;
generating a heating command; and
heating a human body based on the heating command,
wherein the heating command comprises a first heating command generated based on the physiological state information.

In a non-limiting example, the physiological state information comprises first physiological state information, and the method further comprises determining whether to generate the first heating command based on the first physiological state information.

In a non-limiting example, the first physiological state information comprises state information regarding whether a woman is in a menstrual period, and it is determined whether to generate the first heating command based on the state information regarding whether the woman is in the menstrual period.

In a non-limiting example, the physiological state information comprises second physiological state information different from the first physiological state information, the first heating command is generated based on the second physiological state information, and the method further comprises generating a physiological result based on the second physiological state information and determining whether to generate the first heating command based on the physiological result.

In a non-limiting example, the second physiological state information comprises state information regarding whether a woman is in an ovulation period, the physiological result comprises woman menstrual period information, and the woman menstrual period information is generated based on the state information regarding whether the woman is in the ovulation period and it is determined whether to generate the first heating command based on the woman menstrual period information.

In a non-limiting example, the method further comprises steps of:
transmitting the detected physiological state information to an external decision device;
generating a start command based on the transmitted physiological state information; and
receiving the start command and determining whether to generate a second heating command based on the start command.

In a non-limiting example, the step of determining whether to generate the second heating command based on the start command comprises:
generating only one of the first heating command and the second heating command in a preset period of time.

In a non-limiting example, the transmitted physiological state information comprises state information regarding whether a woman is in a menstrual period and/or state information regarding whether a woman is in an ovulation period, and
the step of generating the start command based on the transmitted physiological state information comprises:
determining whether to generate the start command based on the state information regarding whether the woman is in the menstrual period; and/or
generating woman menstrual period information based on the state information regarding whether the woman is in the ovulation period and determining whether to generate the start command based on the woman menstrual period information.

In a non-limiting example, the heating command further comprises a third heating command generated manually.

The physiological adjustment system and the non-claimed physiological adjustment method according to embodiments and examples of the present disclosure can detect physiological state of a user, and heat the body based on the physiological state information of the user, so as to alleviate pain caused by dysmenorrhea, cold uterus and the like of the user. Additionally, the intelligent underpants according to embodiments of the present disclosure act as an intelligent wearable device, improve technological element of the clothes, adjust physiological parameters of the human body by scientific and technological means, and increase additional values of product.

### BRIEF DESCRIPTION OF THE DRAWINGS

Fig. 1 is a schematic functional block diagram of a physiological adjustment system according to an embodiment of the present disclosure;
Fig. 2 is a schematic circuit diagram of a physiological adjustment system according to an embodiment of the present disclosure;
Fig. 3 shows intelligent underpants according to an embodiment of the present disclosure; and
Fig. 4 shows a physiological adjustment method according to an example of the present disclosure.

### DETAILED DESCRIPTION OF PREFERRED EMBODIMENTS

The embodiments of the present disclosure will now be described in detail with reference to the accompanying drawings, in which similar reference numerals refer to similar parts.

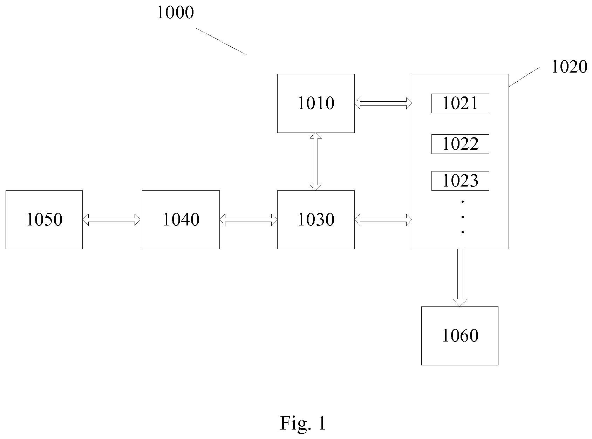

Fig. 1 is a schematic functional block diagram of a physiological adjustment system 1000 according to the present disclosure. The physiological adjustment system 1000 comprises: a heating module 1010 for heating a human body based on a received heating command; a detection module 1020 for detecting a physiological state to obtain physiological state information; and a control module 1030 for sending the heating command to the heating module 1010, wherein the heating command comprises a first heating command generated based on the physiological state information. The physiological adjustment system according to the present disclosure can heat the body based on the physiological state information of a user, so as to alleviate pain caused by dysmenorrhea, cold uterus and the like of the user.

The physiological state information comprises first physiological state information, and the control module is further configured for determining whether to generate the first heating command based on the first physiological state information. The physiological state information comprises state information regarding whether a woman is in a menstrual period, and the control module is further configured for determining whether to generate the first heating command based on the state information regarding whether the woman is in the menstrual period.

In an embodiment of the present disclosure, the detection module 1020 may be arranged on clothes (for example, underpants) and comprise at least one sensor for measuring a physiological index of a human body. The detection module 1020 comprises a blood sensor 1021 capable of detecting blood, and the detection module 1020 obtains state information regarding whether a woman is in a menstrual period through the detection from the blood sensor 1021. The physiological state information comprises state information regarding whether a woman is in a menstrual period, and the control module 1030 is further configured for determining whether to generate the first heating command based on the state information regarding whether the woman is in the menstrual period. It should be noted that the first physiological state information may also comprise other physiological state information as long as it can reflect physiological characteristics of the human body and human discomfort may be alleviated by heating means based on the physiological characteristics.

In an embodiment of the present disclosure, the physiological state information comprises second physiological state information different from the first physiological state information, the first heating command is generated based on the second physiological state information, and the control module is further configured for generating a physiological result based on the second physiological state information and determining whether to generate the first heating command based on the physiological result.

In an embodiment of the present disclosure, the second physiological state information comprises state information regarding whether a woman is in an ovulation period, the physiological result comprises woman menstrual period information, and the control module is further configured for generating the woman menstrual period information based on the state information regarding whether the woman is in an ovulation period and for determining whether to generate the first heating command based on the woman menstrual period information.

In an embodiment of the present disclosure, the detection module 1020 may further comprise an electronic ovulation test strip sensor 1022 capable of detecting a peak level of luteinizing hormone (LH) in residual urine on an underwear. For example, the luteinizing hormone (LH) in the urine appears at a high peak in the first 24 to 48 hours of the ovulation period of the woman, then the detection module 1020 may obtain the state information regarding whether the woman is in an ovulation period based on a detection result from the electronic ovulation test strip sensor 1022.

In an embodiment of the present disclosure, the detection module 1020 further comprises a temperature sensor 1023 capable of measuring a body temperature of the user, so that the user may timely know the body temperature of himself. It will be appreciated by those skilled in the art that the above-described sensors are merely exemplary sensors, but are not intended to limit the present disclosure. The detection module 1020 is not limited to the inclusion of the above-described sensors, but it may include other sensors for measuring specific parameters if required.

In an embodiment of the present disclosure, the control module 1030 may determine whether to generate the first heating command based on the first state information regarding whether the woman is in the menstrual period. In addition, the control module 1030 may generate a physiological result based on the second physiological state information regarding whether the woman is in an ovulation period, the physiological result comprises woman menstrual period information, and the control module 1030 is further configured for determining whether to generate the first heating command based on the woman menstrual period information. Specifically, the menstrual period information includes cycle information of the menstrual period. For example, after obtaining the state information that the woman is in the ovulation period, a starting date of the menstrual period may be determined by adding the ovulation period by about one week, then the cycle of the menstrual period of the woman is a period of 5 to 7 days after the starting date of the menstrual period. In an embodiment of the present disclosure, the control module 1030 determines whether to generate a start command during the menstrual cycle, for example, the start command may be sent out during the menstrual cycle only one time, or the start command may be sent out during the menstrual cycle at intervals. The control module 1030 may not generate any heating command at the end of the cycle of the menstrual period so as to satisfy different individual requirements.

In addition, the physiological adjustment system 100 further comprises a communication module 1040 for receiving a command from an external decision device 1050 and transmitting the physiological state information detected by the detection module 1020 to the external decision device 1050. The transmitted physiological state information comprises state information regarding whether a woman is in a menstrual period and/or state information regarding whether a woman is in an ovulation period, a body temperature of the user, a mood sign indicating physiological state information of the woman and the like. The mood sign may include an unhappy face (for example, a rainy expression) that indicates a woman is in a menstrual period, a face (for example, a smiling face) that indicates the woman is in a safe period, and a face (for example, a small hand) that indicates the woman is in an ovulation period.

The external decision device 1050 may generate a start command based on the transmitted physiological state information. Specifically, the external decision device 1050 determines whether to generate the start command based on the state information regarding whether the woman is in the menstrual period. Optionally, when the woman is in the menstrual period, the start command is generated. Alternatively, the external decision device 1050 generates the woman menstrual period information based on the state information regarding whether the woman is in the ovulation period and determines whether to generate the start command based on the woman menstrual period information. For example, after obtaining the state information that the woman is in the ovulation period, a starting date of the menstrual period may be determined by adding the ovulation period by about one week, then the cycle of the menstrual period of the woman is a period of 5 to 7 days after the starting date of the menstrual period, the external decision device 1050 determines whether to generate the start command during the menstrual cycle, for example, the start command may be sent out during the menstrual cycle only one time, or the start command may be sent out during the menstrual cycle at intervals. The control module 1030 receives the start command and determines whether to generate a second heating command based on the start command. Determining whether to generate the second heating command based on the start command includes generating only one of the first heating command and the second heating command in a preset period of time. In this way, it is possible to prevent the first heating command and the second heating command from repeating, thereby optimizing the system. The external decision device 1050 comprises an intelligent terminal comprising an application program for generating the start command based on the transmitted physiological state information. In this embodiment, the external decision device 1050 may be a smartphone in which an application program for controlling the heating module is installed. The communication module 1040 comprises a wireless communication module, such that it is wirelessly connected to the external decision device 1050, for example via a Bluetooth technology, a WLAN network, an infrared communication technology or the like. The external decision device 1050 may also record and calculate the menstrual cycle of the woman using the physiological state information transmitted by the communication module 1040, and transmit the heating command to the heating module 1010 during the menstrual cycle.

The physiological adjustment system 1000 further comprises a flexible display 1060 connected to the control module 1030 and capable of visually displaying the physiological state information for the user, for example, the body temperature of the user, information regarding whether a woman is in a menstrual period, a mood sign indicating physiological state of the woman, information regarding whether a woman is in an ovulation period and the like. The mood sign may include an unhappy face (for example, a rainy expression) that indicates a woman is in a menstrual period, a face (for example, a smiling face) that indicates the woman is in a safe period, and a face (for example, a small hand) that indicates the woman is in an ovulation period. Of course, the mood sign is not limited to the listed examples, but may include any possible face signs that reflect the physiological state.

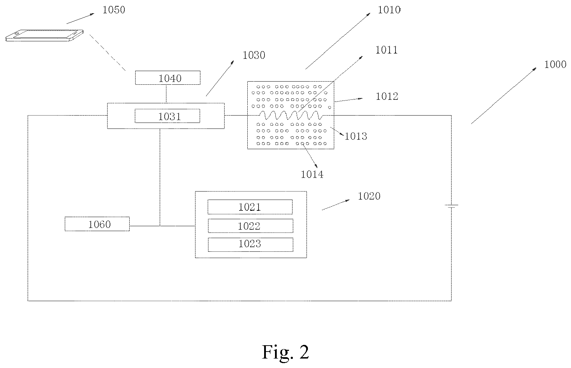

According to an embodiment of the present disclosure, the control module 1030 may further comprise a manual heating switch 1031 (referring to Fig. 2), and the heating command further comprises a third heating command generated by the manual heating switch 1031.

Fig. 2 is a schematic circuit diagram of a physiological adjustment system 1000 according to an embodiment of the present disclosure, in which the principle of a heating circuit is shown predominantly.

In an embodiment of the present disclosure, the heating module 1010 includes an iron-chromium-aluminum alloy heat pipe 1011 functioning as a heating element and a flexible bag container 1012. The iron-chromium-aluminum alloy heat pipe 1011 is arranged in the flexible bag container 1012 filled with liquid essential oil 1013. In an embodiment of the present disclosure, a massage stone 1014 is also provided in the flexible bag container 1012. By means of the essential oil 1013 and the massage stone 1014, it can achieve a uniform heating and massage effect. It should be understood that the essential oil 1013 and the massage stone 1014 themselves may be made from insulation materials, and the flexible bag container 1012 may also be made of an insulation material. When the physiological adjustment system 1000 is applied to a woman's underpants, it is easily to be worn and can alleviate the dysmenorrhea symptom of the woman.

The blood sensor 1021, the electronic ovulation test strip sensor 1022 and the temperature sensor 1023 can respectively detect the blood, the peak level of the luteinizing hormone (LH) in the residual urine on the underwear and the human body temperature, so that it can obtain the physiological state information regarding whether the woman is in the menstrual period and whether the woman is in the ovulation period and the body temperature of the woman. The flexible display 1060 is configured for visually displaying the physiological state information for the user, such as, the body temperature of the user, information regarding whether a woman is in a menstrual period, a mood sign indicating physiological state of the woman, information regarding whether a woman is in an ovulation period and the like. The mood sign may include an unhappy face (for example, a rainy expression) that indicates a woman is in a menstrual period, a face (for example, a smiling face) that indicates the woman is in a safe period, and a face (for example, a small hand) that indicates the woman is in an ovulation period.

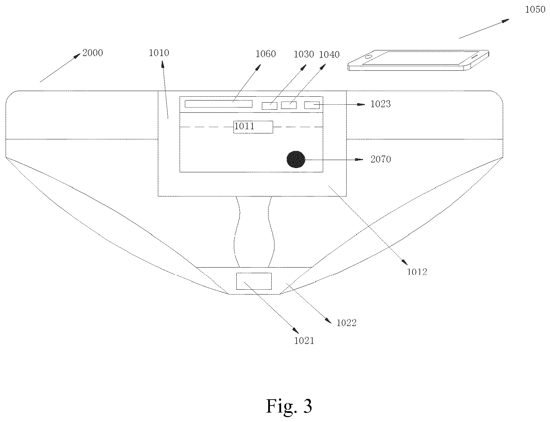

Fig. 3 shows intelligent underpants 2000 according to an embodiment of the present disclosure. The intelligent underpants 2000 comprises the above physiological adjustment system. The heating module 1010 is arranged at a position corresponding to a lower abdomen of a wearer on a front side of the underpants 2000. The heating module 1010 adopts an iron-chromium-aluminum alloy heat pipe 1011 to act as a heating element. The iron-chromium-aluminum alloy heat pipe 1011 is arranged in a flexible bag container 1012 filled with liquid essential oil and a massage stone.

The detection module 1020 includes a blood sensor 1021, an electronic ovulation test strip sensor 1022, and a temperature sensor 1023. The blood sensor 1021 is arranged at a crotch portion of the underpants 2000 close to the lower abdomen portion.

The electronic ovulation test strip sensor 1022 is arranged at the crotch portion of the underpants and detects a peak level of luteinizing hormone in residual urine to further obtain the state information regarding whether the woman is in the ovulation period, and displays it by the flexible display 1060, thereby assisting the woman in preparing for pregnancy and increasing chance of pregnancy. The temperature sensor 1023 is capable of measuring the abdomen temperature of the wearer.

The communication module 1040 may be arranged at an upper side of the underpants 2000 close to a waist to facilitate communicating with the external smartphone 1050. It should be understood that the communication can be implemented as long as the communication module 1040 is arranged on the underpants 2000, therefore, the detailed arrangement position will not be limited in the embodiments of the present disclosure.

The flexible display 1060 is also arranged on the upper side of the underpants 2000 close to the waist on the front side, and configured for visually displaying the physiological state information for the user, such as, the body temperature of the user, information regarding whether a woman is in a menstrual period, a mood sign indicating physiological state of the woman, information regarding whether a woman is in an ovulation period and the like. The mood sign may include an unhappy face (for example, a rainy expression) that indicates a woman is in a menstrual period, a face (for example, a smiling face) that indicates the woman is in a safe period, and a face (for example, a small hand) that indicates the woman is in an ovulation period.

The control module 1030 and its manual heating switch 1031 are provided on the front side of the underpants 2000 for ease of operation.

The intelligent underpants 2000 can also be provided with an indicator light 2070. The indicator light 2070 may be a color-changeable LED lamp so as to indicate the relevant working status information of the underpants. For example, a flashing red light indicates lack of electricity, a flashing green light indicates that the underpants are in an heating status. It will be understood by those skilled in the art that the indicator light may work according to a presetting such that different lighting statuses indicate different working status information, and the above-described working status information is merely exemplary, and it is possible to set the working status information of the indicator light by those skilled in the art as required.

The intelligent underpants 2000 according to the present disclosure may be dry-cleaned or brushed by using detergent.

It will be appreciated by those skilled in the art that the positions of the various components of the intelligent underpants 2000 are exemplary, but not restrictive. The above-described components may be adjusted according to requirements or design preferences, without adversely affecting the implementation of their functionality.

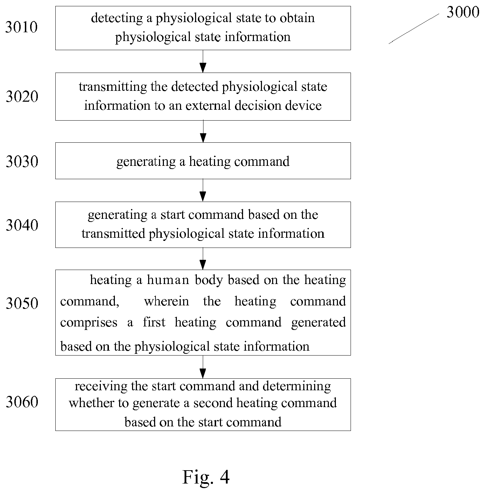

Fig. 4 shows a physiological adjustment method 3000 according to a non-claimed example of the present disclosure. The physiological adjustment method comprises following steps:
step 3010: detecting a physiological state to obtain physiological state information;
step 3030: generating a heating command; and
step 3050: heating a human body based on the heating command, wherein the heating command comprises a first heating command generated based on the physiological state information.

According to an example of the present disclosure, the physiological state information comprises first physiological state information, and the method further comprises a step of determining whether to generate the first heating command based on the first physiological state information. The first physiological state information comprises state information regarding whether a woman is in a menstrual period, and it is determined whether to generate the first heating command based on the state information regarding whether the woman is in the menstrual period.

The physiological state information further comprises second physiological state information different from the first physiological state information, the first heating command is generated based on the second physiological state information, and the method further comprises a step of generating a physiological result based on the second physiological state information and determining whether to generate the first heating command based on the physiological result. The second physiological state information comprises state information regarding whether a woman is in an ovulation period, the physiological result comprises woman menstrual period information, and the woman menstrual period information is generated based on the state information regarding whether the woman is in the ovulation period and it is determined whether to generate the first heating command based on the woman menstrual period information.

The method further comprises following steps:
step 3020: transmitting the detected physiological state information to an external decision device;
step 3040: generating a start command based on the transmitted physiological state information; and
step 3060: receiving the start command and determining whether to generate a second heating command based on the start command.

The step 3060 of determining whether to generate the second heating command based on the start command comprises: generating only one of the first heating command and the second heating command in a preset period of time. That is to say, the step 3010, the step 3030, the step 3050 and the steps 3020, 3040, 3060 may be performed in parallel, and have no interference with each other, and the step number does not mean the relevant steps must be performed in this order. The sequence of the steps in this embodiment is merely exemplary, but not restrictive, and the steps may be adjusted in the aspect of sequence according to the actual situation. However, only one of the first heating command and the second heating command is generated in a preset period of time, that is, only one heating command is generated in the preset period of time.

The transmitted physiological state information comprises state information regarding whether a woman is in a menstrual period and/or state information regarding whether a woman is in an ovulation period, and
the step of generating the start command based on the transmitted physiological state information comprises:
determining whether to generate the start command based on the state information regarding whether the woman is in the menstrual period; and/or
generating woman menstrual period information based on the state information regarding whether the woman is in the ovulation period and determining whether to generate the start command based on the woman menstrual period information. The menstrual period information includes cycle information of the menstrual period.

The heating command further comprises a third heating command generated manually.

## Claims

1. A physiological adjustment system (1000), comprising:
a heating module (1010) for heating a human body based on a received heating command;
a detection module (1020) for detecting a physiological state to obtain physiological state information, said physiological state including a menstrual period; and
a control module (1030) for sending the heating command to the heating module (1010), wherein the heating command comprises a first heating command generated based on the physiological state information,
the heating module (1010) is adapted to heat the lower abdomen of a user,
the physiological state information comprises first physiological state information,
the first physiological state information comprises state information regarding whether a woman is in a menstrual period, and the control module (1030) is further configured for determining whether to generate the first heating command based on the state information regarding whether the woman is in the menstrual period,
**characterized in that**,
the detection module (1020) comprises a blood sensor (1021) for detecting blood to obtain state information regarding whether the woman is in the menstrual period.

2. The physiological adjustment system (1000) according to claim 1, wherein the physiological state information comprises second physiological state information different from the first physiological state information, the first heating command is generated based on the second physiological state information, and the control module (1030) is further configured for generating a physiological result based on the second physiological state information and determining whether to generate the first heating command based on the physiological result.

3. The physiological adjustment system (1000) according to claim 2, wherein the second physiological state information comprises state information regarding whether a woman is in an ovulation period, the physiological result comprises woman menstrual period information, and the control module (1030) is further configured for generating the woman menstrual period information based on the state information regarding whether the woman is in the ovulation period and determining whether to generate the first heating command based on the woman menstrual period information.

4. The physiological adjustment system (1000) according to claim 1, further comprising a communication module (1040) for receiving a command from an external decision device (1050) and transmitting the physiological state information detected by the detection module (1020) to the external decision device (1050), wherein the external decision device (1050) is configured for generating a start command based on the transmitted physiological state information, and the control module (1030) is configured for receiving the start command and determining whether to generate a second heating command based on the start command.

5. Intelligent underpants (2000), comprising the physiological adjustment system (1000) according to any one of claims 1-4, wherein the heating module (1010) is arranged at a position corresponding to a lower abdomen of a wearer on a front side of the underpants (2000).

6. The intelligent underpants (2000) according to claim 5, further comprising an insulating bag filled with essential oil (1013), and a heat generation source of the heating module (1010) is provided in the essential oil (1013), and/or
wherein the detection module (1020) comprises an electronic ovulation test strip sensor (1022) provided in a crotch portion of the intelligent underpants (2000), and the electronic ovulation test strip sensor (1022) is configured for detecting a peak level of luteinizing hormone in residual urine to obtain state information regarding whether a woman is in an ovulation period, and/or
wherein the physiological adjustment system (1000) further comprises a temperature sensor (1023) for measuring an abdomen temperature of the wearer.

## Patentansprüche

1. Physiologisches Einstellungssystem (1000), umfassend:
ein Heizmodul (1010) zum Erwärmen eines menschlichen Körpers basierend auf einem empfangenen Heizbefehl;
ein Detektionsmodul (1020) zum Detektieren eines physiologischen Zustands, um eine physiologische Zustandsinformation zu erhalten, wobei der physiologische Zustand eine Menstruationsperiode umfasst; und
ein Steuermodul (1030) zum Senden des Heizbefehls an das Heizmodul (1010), wobei der Heizbefehl einen ersten Heizbefehl umfasst, der basierend auf der physiologischen Zustandsinformation erzeugt wurde,
wobei das Heizmodul (1010) ausgelegt ist, den unteren Abdomen eines Benutzers zu erwärmen,
wobei die physiologische Zustandsinformation eine erste physiologische Zustandsinformation umfasst,
wobei die erste physiologische Zustandsinformation eine Zustandsinformation in Bezug darauf, ob sich eine Frau in einer Menstruationsperiode befindet, umfasst und das Steuermodul (1030) ferner ausgebildet ist, zu bestimmen, ob der erste Heizbefehl basierend auf der Zustandsinformation in Bezug darauf, ob sich die Frau in der Menstruationsperiode befindet, erzeugt werden soll,
**dadurch gekennzeichnet, dass**
das Detektionsmodul (1020) einen Blutsensor (1021) zum Detektieren von Blut umfasst, um eine Zustandsinformation in Bezug darauf, ob sich die Frau in der Menstruationsperiode befindet, zu erhalten.

2. Physiologisches Einstellungssystem (1000) nach Anspruch 1, wobei die physiologische Zustandsinformation eine zweite physiologische Zustandsinformation umfasst, die sich von der ersten physiologischen Zustandsinformation unterscheidet, wobei der ersten Heizbefehl basierend auf der zweiten physiologischen Zustandsinformation erzeugt wird, und das Steuermodul (1030) ferner zum Erzeugen eines physiologischen Ergebnisses basierend auf der zweiten physiologischen Zustandsinformation und Bestimmen, ob der erste Heizbefehl basierend auf dem physiologischen Ergebnis erzeugt werden soll, ausgebildet ist.

3. Physiologisches Einstellungssystem (1000) nach Anspruch 2, wobei die zweite physiologische Zustandsinformation eine Zustandsinformation in Bezug darauf, ob sich eine Frau in einer Ovulationsperiode befindet, umfasst, wobei das physiologische Ergebnis eine Menstruationsperiodeninformation einer Frau umfasst, und wobei das Steuermodul (1030) ferner zum Erzeugen der Menstruationsperiodeninformation der Frau basierend auf der Zustandsinformation in Bezug darauf, ob sich die Frau in der Ovulationsperiode befindet, und Bestimmen, ob der erste Heizbefehl basierend auf der Menstruationsperiodeninformation der Frau erzeugt werden soll, ausgebildet ist.

4. Physiologisches Einstellungssystem (1000) nach Anspruch 1, ferner umfassend ein Kommunikationsmodul (1040) zum Empfangen eines Befehls von einer externen Entscheidungsvorrichtung (1050) und Übertragen der durch das Detektionsmodul (1020) detektierten physiologischen Zustandsinformation an die externe Entscheidungsvorrichtung (1050), wobei die externe Entscheidungsvorrichtung (1050) zum Erzeugen eines Startbefehls basierend auf der übertragenen physiologischen Zustandsinformation ausgebildet ist, und das Steuermodul (1030) zum Empfangen des Startbefehls und Bestimmen, ob ein zweiter Heizbefehl basierend auf dem Startbefehl erzeugt werden soll, ausgebildet ist.

5. Intelligente Unterhose (2000), umfassend ein physiologisches Einstellungssystem (1000) nach einem der Ansprüche 1-4, wobei das Heizmodul (1010) an einer Position angeordnet ist, die einem unteren Abdomen eines Trägers an einer Vorderseite der Unterhose (2000) entspricht.

6. Intelligente Unterhose (2000) nach Anspruch 5, ferner umfassend eine isolierende Tasche, die mit ätherischem Öl (1013) gefüllt ist, und wobei eine Wärmeerzeugungsquelle des Heizmoduls (1010) in dem ätherischen Öl (1013) bereitgestellt ist, und/oder
wobei das Detektionsmodul (1020) einen elektronischen Ovulationsteststreifensensor (1022) umfasst, der in einem Zwickelabschnitt der intelligenten Unterhose (2000) bereitgestellt ist, und der Ovulationsteststreifensensor (1022) zum Detektieren eines Spitzenspiegels eines luteinisierenden Hormons in Restharn ausgebildet ist, um eine Zustandsinformation in Bezug darauf, ob sich eine Frau in einer Ovulationsperiode befindet, zu erhalten, und/oder
wobei das physiologische Einstellungssystem (1000) ferner einen Temperatursensor (1023) zum Messen einer Abdomentemperatur des Trägers umfasst.

## Revendications

1. Système de régulation physiologique (1000), comprenant :
un module chauffant (1010) destiné à chauffer un corps humain sur la base d'une commande de chauffage reçue ;
un module de détection (1020) destiné à détecter un état physiologique pour obtenir des informations d'état physiologique, ledit état physiologique comprenant une période menstruelle ; et
un module de commande (1030) destiné à envoyer la commande au module chauffant (1010), dans lequel la commande chauffante comprend une première commande chauffante générée sur la base des informations d'état physiologique,
le module chauffant (1010) est conçu pour chauffer la partie inférieure de l'abdomen d'une utilisatrice,
les informations d'état physiologique comprennent des premières informations d'état physiologique,
les premières informations d'état physiologique comprennent des informations d'état permettant de savoir si oui ou non une femme est dans une période menstruelle, et le module de commande (1030) est en outre configuré pour déterminer s'il convient de générer la première commande de chauffage sur la base des informations d'état permettant de savoir si oui ou non la femme est dans la période menstruelle,
**caractérisé en ce que**,
le module de détection (1020) comprend un capteur de sang (1021) destiné à détecter du sang afin d'obtenir des informations d'état permettant de savoir si oui ou non la femme est dans la période menstruelle.

2. Système de régulation physiologique (1000) selon la revendication 1, dans lequel les informations d'état physiologique comprennent des secondes informations d'état physiologique différentes des premières informations d'état physiologique, la première commande de chauffage est générée sur la base des secondes informations d'état physiologique, et le module de commande (1030) est en outre configuré pour générer un résultat physiologique sur la base des secondes informations d'état physiologique et pour déterminer s'il convient de générer la première commande de chauffage sur la base du résultat physiologique.

3. Système de régulation physiologique (1000) selon la revendication 2, dans lequel les secondes informations d'état physiologique comprennent des informations d'état permettant de savoir si oui ou non une femme est dans une période d'ovulation, le résultat physiologique comprend des informations de période menstruelle féminine, et le module de commande (1030) est en outre configuré pour générer les informations de période menstruelle féminine sur la base des informations d'état permettant de savoir si oui ou non la femme est dans la période ovulation et pour déterminer s'il convient de générer la première commande de chauffage sur la base des informations de période menstruelle féminine.

4. Système de régulation physiologique (1000) selon la revendication 1, comprenant en outre un module de communication (1040) destiné à recevoir une commande en provenance d'un dispositif de décision externe (1050) et à émettre les informations d'état physiologique détectées par le module de détection (1020) vers le dispositif de décision externe (1050), dans lequel le dispositif de décision externe (1050) est configuré pour générer une commande de démarrage sur la base des informations d'état physiologique émises, et le module de commande (1030) est configuré pour recevoir la commande de démarrage et pour déterminer s'il convient de générer une seconde commande de chauffage sur la base de la commande de démarrage.

5. Sous-vêtements intelligents (2000), comprenant le système de régulation physiologique (1000) selon l'une quelconque des revendications 1 à 4, dans lequel le module chauffant (1010) est placé en une position correspondant à la partie inférieure de l'abdomen d'une utilisatrice sur un côté avant des sous-vêtements (2000) .

6. Sous-vêtements intelligents (2000) selon la revendication 5, comprenant en outre un sac isolant rempli d'huile essentielle (1013), et une source de production de chaleur du module chauffant (1010) est placée dans l'huile essentielle (1013), et/ou
dans lesquels le module de détection (1020) comprend un capteur de bandelette de test d'ovulation électronique (1022) situé dans une partie entrejambe des sous-vêtements intelligents (2000), et le capteur de bandelette de test d'ovulation électronique (1022) est configuré pour détecter un niveau maximum d'hormone de lutéinisation dans l'urine résiduelle afin d'obtenir des informations d'état permettant de savoir si oui ou non une femme est dans une période d'ovulation, et/ou
dans lequel le système de régulation physiologique (1000) comprend en outre un capteur de température (1023) destiné à mesurer une température abdominale de l'utilisatrice.
